# EUROPÄISCHE PATENTSCHRIFT

(11) **EP 1 499 564 B1**
(45) Veröffentlichungstag und Bekanntmachung des Hinweises auf die Patenterteilung: **24.06.2009**
(21) Anmeldenummer: 03718760.6
(22) Anmeldetag: 17.04.2003
(51) Int. Cl.: C02F 1/50

(54) **VERWENDUNG EINES ENTKEIMUNGSSYSTEMS ZUR ENTKEIMUNG VON TRINKWASSER**
USE OF A SYSTEM FOR STERILISING DRINKING WATER
UTILISATION D'UN SYSTEME DE STERILISATION D'EAU POTABLE

(30) Priorität: 19.04.2002 DE 10217649
(43) Veröffentlichungstag der Anmeldung: 26.01.2005
(73) Patentinhaber: PaSta Aktiengesellschaft, 9490 Vaduz (LI)
(72) Erfinder: Stadelmann, Heinz W., 9442 Berneck (CH)
(74) Vertreter: Mammel, Ulrike
(86) Internationale Anmeldenummer: PCT/EP2003/003917
(87) Internationale Veröffentlichungsnummer: WO 2003/089377

(56) Entgegenhaltungen:
- EP-A- 0 911 297
- WO-A-00/56371
- AU-B- 504 777
- DE-A- 10 029 082
- US-A- 4 915 955
- PATENT ABSTRACTS OF JAPAN vol. 1996, no. 07, 31. Juli 1996 (1996-07-31) & JP 08 071339 A (MITSUBISHI MATERIALS CORP), 19. März 1996 (1996-03-19)

## Beschreibung

Mikrobiologisch belastetes Trinkwasser kann beim Genuss zu schweren Erkrankungen führen. Um dies zu verhindern, wird Trinkwasser durch entsprechende physikalisch-chemische Prozesse aufbereitet. Um einen nachhaltigen Schutz gegen Rückverkeimung zu gewährleisten, wird dem aufbereiteten Trinkwasser zum Beispiel Chlor zugesetzt.

Lässt man Trinkwasser über längere Zeit stehen oder bereitet man das von den Versorgern über das öffentliche Leitungsnetz zur Verfügung gestellte Trinkwasser durch Aktivkohlefilterung oder durch umgekehrte Osmose am point of use zur Geschmacksverbesserung oder zur weiteren Reduktion unerwünschter Inhaltsstoffe nachträglich auf, dann geht dieser Rückverkeimungsschutz durch die Entfernung des im Wasser befindlichen Chlors verloren. So ist bekannt, dass die vorwiegend im häuslichen Bereich eingesetzten Filtersysteme zur Trinkwassernachbehandlung innerhalb kurzer Zeit derart verkeimen, dass zum Beispiel die Richtwerte der Trinkwasserverordnung deutlich überschritten werden und dieses Wasser aus mikrobiologischen Gründen für den menschlichen Verzehr somit ungeeignet ist. Gleiches gilt für in Tanks abgefülltes Trinkwasser (zum Beispiel Wohnmobile) oder für Luftbefeuchter und Klimaanlagen, wo sich oft innerhalb weniger Tage eine starke Verkeimung beobachten lässt.

Diese Keime werden beim Trinken aufgenommen oder mit dem verdunsteten Wasser freigesetzt. Im letzteren Fall kann das zum sogenannten Befeuchterfieber führen. Setzt man als Verkeimungsschutz biozide Wirkstoffe zu, dann werden diese beim Trinken oder über die Atmung aufgenommen. Dies kann ernsthafte Erkrankungen zur Folge haben.

In geschlossenen Systemen wie Tanks, Leitungssystemen oder Wasserbetten verändert sich aufgrund einsetzender mikrobiologischer Aktivitäten die Wasserqualität. Das Wasser riecht muffig, die in Wasserbetten beobachtete Gasentwicklung macht einen regelmäßigen Austausch des Wassers erforderlich.

Weiterhin kritisch sind Warmwasserbehälter, Duschköpfe oder einfache Untertischboiler, in denen sich aufgrund der erhöhten Temperatur besonders gefährliche, thermotolerante Keimbelastungen entwickeln können.

Hygienisch einwandfreies Trinkwasser ist eines der dringendsten Probleme der Entwicklungsländer. Die in der "Ersten Welt" eingesetzten Technologien zur Trinkwasserdesinfektion sind teuer und müssen laufend gewartet und kontrolliert werden, um eine zuverlässige Entkeimung des Trink- und Brauchwassers sicherzustellen. Die aufwendige Steuer-, Mess- und Regeltechnik ist zudern nur von qualifiziertem Fachpersonal beherrschbar. Kostengünstig können diese Anlagen nur als zentrale Anlage betrieben werden, was eine entsprechende Infrastruktur voraussetzt. Diese Voraussetzungen sind in der "Dritten Welt" nicht gegeben.

Für die "Dritte Welt" müssten vielmehr Entkeimungsverfahren bereitgestellt werden, die über lange Zeiträume (Jahre) wartungsfrei eine zuverlässige Entkeimung des Trink- und Brauchwassers sicherstellen.

Bei den bislang eingesetzten Technologien wird eine zuverlässige Entkeimung nur dann erreicht, wenn eine kontinuierliche Überwachung der relevanten Betriebsparameter erfolgt, gleichzeitig eine permanente Betreuung durch qualifiziertes Fachpersonal gewährleistet ist und man über eine funktionierende Infrastruktur - Leitungsnetz - verfügt. Diese Voraussetzungen sind insbesondere in der "Dritten Welt" und im häuslichen Bereich nicht oder nur unvollständig gegeben. Die hohen Kosten und/oder der Mangel an qualifiziertem Personal sprechen hier dagegen.

Aufgrund der oben geschilderten Probleme ist das Trinkwasser in großen Teilen der so genannten "Dritten Welt" erheblich mikrobiell belastet.

Im häuslichen Bereich ("Erste Welt") werden verschiedene Nachbehandlungsanlagen eingesetzt, die auf photochemischer Behandlung des Wassers (UV-Desinfektion) oder chemischen Zusätzen, beispielsweise Silberverbindungen, basieren.

Eine Desinfektion mit UV-Licht findet jedoch nur während des Bestrahlungsvorgangs selbst statt, so dass keine "Depot-Wirkung" besteht. Darüber hinaus führt die Bestrahlung mit UV-Licht zur unkontrollierten photochemischen Veränderung der Wasserinhaltsstoffe, was unerwünscht ist.

Neben der UV-Desinfektion sind auch verschiedene Entkeimungsverfahren auf Silberbasis bekannt.

Die WO 82/03381 beschreibt ein Verfahren zur Herstellung von Formkörpern zur Keimfreihaltung von Wässern und wässrigen Lösungen, bei dem eine in Wasser schwerlösliche, die Entwicklung von Keimen verhindernde Metallverbindung wie beispielsweise AgCl mit oder ohne Trägersubstanzen unter einem Druck von mindestens 8.000 kg/cm² verformt wird. Anschließend wird die Metallverbindung durch Beimengung leichtlöslicher Salze, durch thermische oder chemische Behandlung aktiviert.

Als Formkörper werden Presslinge, in eine Kunststoffmembran eingeschweißte Silberverbindungen oder auf einem inerten Träger, zum Beispiel Silber-, Gold- oder Platindraht, Magnesiumoxid, Aluminiumoxid aufgeschmolzene Metallverbindungen eingesetzt.

Die gewünschte Keimfreiheit wird bei diesen in geschossenen Systemen eingesetzten Formkörpern, die 0,1 bis 1,0 mg Silber/l freisetzen, durch einen Silberüberschuss erreicht. Diese Silberwerte liegen weit über der derzeit zulässigen Silberkonzentration von 0,01 mg/l (allgemeiner Wert EU-Richtlinie, Trinkwasser-Verordnung (TVO), beziehungsweise 0,08 mg/l (TVO bei Behandlung mit Silber)) und überschreiten teilweise selbst die zulässigen Silberwerte in Abwässern, die derzeit, abhängig von Industriezweig, bei 0,1, 0,5 beziehungsweise 0,7 mg/l liegen.

Nachteilig an diesen Formkörpern ist weiterhin neben der aufwendigen Herstellung unter Druck, dass diese Formkörper nur in geschlossenen Systemen einsetzbar sind und nach Öffnen des Systems nachdosiert werden muss. Die in dieser Druckschrift vorgeschlagenen Aluminiumverbindungen als Trägermaterialien sind zudem hinsichtlich der Freisetzung von Al³⁺-Ionen bedenklich, da sie toxikologisch relevant sein können. Auch können die aus dieser Druckschrift bekannten Formkörper nicht zur Keimfreihaltung von Wässern und wässrigen Lösungen eingesetzt werden, die unedlere Metalle als Silber enthalten und müssen im Dunkeln aufbewahrt werden.

Die oben beschriebenen Probleme mit der Überschreitung der zulässigen Silberionenkonzentrationen, dem unkontrollierten Freisetzen von Silberionen und dem Erfordernis der Nachdosierung treten darüber hinaus auch in dem aus dem Stand der Technik zur Entkeimung bekannten Zutropfen von Silbersalzlösungen oder dem Einsatz von mit Silberionen beschichteter Aktivkohle oder den Silberionen umfassenden Ionenaustauschern auf.

Aus der DE 712 792 sind Verfahren zur Herstellung von Erzeugnissen gesteigerter oligodynamischer Wirksamkeit bekannt, bei welchen angeätzte Edelmetalle zur Sterilisation verwendet werden. Das Ätzen erfolgt unter vergleichsweise milden Bedingungen in Wasserstoffperoxidlösung, Hypochloridlösung oder elektrolytisch mit geringen Stromdichten, um die Edelmetalloberfläche aufzurauen. Die Behandlung mit diesen Ätzmitteln dient dazu, im wesentlichen an der Edelmetalloberfläche adsorbierte organische Moleküle zu entfernen. Der angeätzte Grundkörper wird anschließend direkt zur Sterilisation eingesetzt.

Aus der DE 2 307 345 ist bekannt, zur Desinfektion von Wasser einen Filterkörper aus Kupfernetz, der stellenweise versilbert ist, einzusetzen.

Aus der DE 100 29 082 ist weiterhin ein Verfahren zur Entkeimung von Trinkwasser und Brauchwasser durch aktivierte Edelmetalle bekannt, bei dem ein Edelmetall in einem ersten Schritt zur Entfernung von Passivierungsschichten angeätzt und anschließend in einer nicht näher bezeichneten Salzlösung aktiviert wird.

Die Aufgabe der vorliegenden Erfindung besteht darin, Entkeimungssysteme bereitzustellen, die einfach herstellbar und umweltfreundlich sind sowie über lange Zeiträume (Jahre) wartungsfrei direkt am point of use eine zuverlässige Entkeimung des Trinkwassers sicherstellen.

Diese Aufgabe wird durch die Merkmale des Anspruchs 1 gelöst.

Zur Herstellung des erfindungsgemäßen Entkeimungssystems wird die Edelmetalloberfläche eines Edelmetall umfassenden Basismaterials zunächst in einer sauren Lösung oxidiert und direkt anschließend oder nach Abspülen mit Wasser in einer wässrigen Salzlösung behandelt. Durch die Behandlung mit der wässrigen Salzlösung werden in Abhängigkeit von der Art und Konzentration der Anionen in der wässrigen Salzlösung schwerlösliche Edelmetallsalze oder ein Gemisch von Edelmetallsalzen unterschiedlicher Löslichkeitsprodukte auf der Edelmetalloberfläche gebildet, die direkt auf dem Edelmetallmaterial aufgewachsen sind. Hierdurch werden wesentliche Vorteile erreicht:

Das erfindungsgemäße Entkeimungssystem ist selbsterneuernd, da die bei der Entkeimung an das Wasser abgegebenen Edelmetallionen aus der Oberfläche direkt aus dem Kernmaterial nachgeliefert werden.

Zwischen Kernmaterial und Edelmetallsalz-Oberfläche existiert keine Grenzschicht. Die Erneuerung verbrauchter Edelmetallionen aus dem Kernmaterial ist somit nicht kinetisch gehemmt, so dass eine hinreichend schnelle Nachlieferung durch das Redox-System Edelmetall/Edelmetallion gewährleistet ist. Der zur Oxidation notwendige Sauerstoff kann dank der mikroporösen, amorphen Oberfläche ungehindert an die Ag/Ag⁺-Grenzfläche diffundieren.

Die Edelmetallsalz-Oberfläche ist extrem groß und weist mit amorphen und kristallinen Bereichen Regionen unterschiedlicher Aktivität auf. Die kristallinen und amorphen Edelmetallsalze stehen im direkten Kontakt zueinander und im direkten Kontakt zur blanken Silberoberfläche, so dass sich insgesamt folgende Regionen unterschiedlicher Redoxaktivität und Löslichkeit ausbilden:
i) über Poren der amorphen Schicht zugängliche reine Silberoberfläche,
ii) amorphe Edelmetallsalzschicht,
iii) kristalline Edelmetallsalzschicht,
iv) Kontaktzone Silber - amorphe Edelmetallsalzschicht,
v) Kontaktzone Silber - kristalline Edelmetallsalzschicht und
vi) Kontaktzone amorphe Edelmetallsalzschicht - kristalline Edelmetallsalzschicht,
vii) Kontaktzone Silber - amorphe Edelmetallsalzschicht - kristalline Edelmetallsalzschicht,
also ein-, zwei- beziehungsweise dreiphasige Gebiete, deren Verhältnis sich durch die Herstellungsbedingungen steuern lässt.

Befindet sich das erfindungsgemäße Entkeimungssystem in dem zu entkeimenden Wasser, so stellt sich ein Gleichgewicht der Edelmetallionen im Wasser und auf der Oberfläche ein. Die gelösten Ionen üben einen "Druck" auf die Ionen der Oberfläche aus und verhindern somit, dass diese ebenfalls in Lösung gehen. Bei der Entnahme von Wasser und dem dadurch im allgemeinen bedingten Zustrom von frischem Wasser im offenen System ändert sich die Edelmetallionenkonzentration und damit der "Druck" auf die Ionen der Oberfläche. Diese gehen so lange in Lösung, bis die ursprüngliche Konzentration wieder hergestellt ist. Die Verluste von Edelmetallionen an der Oberfläche werden durch das Edelmetall-Basismaterial ausgeglichen.

Durch das Redox-System Edelmetall/Edelmetallsalz (kinetisch nicht gehemmt) ist das System, auch bei Einsatz von Silbersalzen, gegen Belichtung und unedlere Metallionen als das betreffende Edelmetallsalz unempfindlich.

Als Basismaterial kommen Edelmetalle beziehungsweise Edelmetalllegierung auf Silber-, Kupfer- und Goldbasis in Form massiver Körper, Drähte, Metallwollen, verwobener oder gestrickter Drähte beziehungsweise Edelmetalldraht/Kunststoff- und/oder Edelmetalldraht/Kohlefaserfaden-Mischgewebe oder mit diesen Edelmetallen beschichtete Träger, Edelmetallpulver, Edelmetallpulver umfassende Formkörper sowie Edelmetallpulver umfassende Beschichtungen in Frage.

Erfindungsgemaß wird Silber als Edelmetall verwendet.

Weiterhin ist möglich, als Basismaterial Edelmetallpulver einzusetzen. Die Edelmetallpulver, erfindungsgemäß Silberpulver, können Korngrößen vom Nanometerbereich bis in den Korngrößenbereich von Pulverlacken aufweisen, bevorzugt sind Korngrößen im Bereich von etwa 1 bis 500 µm, besonders bevorzugt von etwa 10 bis 90 µm.

Die Oxidation der Edelmetalloberfläche erfolgt in einer sauren, vorzugsweise Wasser umfassenden Lösung. Vorzugsweise umfasst die Oxidationslösung oxidierende anorganische oder organische Säuren und/oder Mischungen oxidierender anorganischer oder organischer Säuren.

Vorzugsweise umfassen die Säuren Schwefel in der Oxidationsstufe + 6, Stickstoff in der Oxidationsstufe + 5, die Halogene Chlor, Brom und Iod in der Oxidationssture + 5 oder + 7, Bor in der Oxidationsstufe + 3 und/oder Sauerstoff in der Oxidationsstufe - 1 oder - 2.

Auch der Einsatz von wässrigen Lösungen mit wasserlöslichen stark oxidierenden Substanzen und Säuren ist möglich.

Der pH-Wert der Oxidationslösung sollte vorzugsweise kleiner 1 ein.

Die chemische Zusammensetzung der sauren Oxidationslösung und die Oxidationsbedingungen sind von der Art der eingesetzten Edelmetalle abhängig. In Abhängigkeit von dem jeweiligen Oxidationsmittel kann bereits bei Raumtemperatur die gewünschte Oxidation des Edelmetalls erzielt werden. Die Säuren beziehungsweise Oxidationsmittel werden vorzugsweise in einer Konzentration zwischen 0,1 Gew.% und einer konzentrierten Lösung, besonders bevorzugt in einer 25 bis 30 Gew.% Lösung bei Edelmetalldrähten und 10 bis 18 Gew.% bei Edelmetallpulvern, eingesetzt.

Durch die Oxidation der Edelmetalloberfläche wird nicht nur die elementare Edelmetalloberfläche unter Bildung positiv geladener Edelmetallionen umgewandelt, sondern es werden auch vorhandene Passivierungsschichten und organische Verunreinigungen entfernt.

Als Gegenionen der Edelmetallionen auf der Oberfläche dienen zunächst im wesentlichen die Anionen der Säuren beziehungsweise die Oxoanionen der Oxidationsmittel. Diese Gegenionen werde dann entsprechend der Zusammensetzung des Salzbades und dem gegebenenfalls vorangehenden Spülen mit Wasser unter Bildung schwerlöslicher Edelmetallsalze oder einem Gemisch von Edelmetallsalzen unterschiedlicher Löslichkeitsprodukte durch die Anionen der Salzlösung ersetzt.

Um die Oxidation des Edelmetalls auf den Oberflächenbereich zu begrenzen, Edelmetallverluste zu vermeiden und die mechanische Stabilität zu erhalten, wird die Verweildauer im Oxidationsbad auf wenige Sekunden bis Minuten beschränkt. Es hat sich als vorteilhaft erwiesen, den Oxidationsprozess anschließend schlagartig durch Eintauchen oder Spülen mit neutralem, Oxidationsmittel freiem Leitungswasser bei Raumtemperatur zu unterbrechen. Durch das Spülen beziehungsweise Eintauchen in Leitungswasser wird nicht nur die Oxidation unterbrochen, sondern die durch die Oxidation gebildeten leichtlöslichen Edelmetallsalze werden auch wenigstens teilweise durch die in dem Leitungswasser enthaltenen Hydroxid- und Carbonationen in Edelmetallhydroxide, -oxide und/oder - carbonate umgewandelt. Auch werden Kosten im Zusammenhang mit der Verunreinigung der Salzlösung durch Einschleppen der Oxidationslösung vermieden.

Nach dem Spülen mit Leitungswasser oder direkt nach der Behandlung des Basismaterials in dem Oxidationsmittel wird das an der Edelmetalloberfläche oxidierte Basismaterial in eine Salzlösung getaucht.

Die Salzlösung umfaßt Chlorid. Durch die Art und die Konzentrationsverhältnisse der betreffenden Anionen in der salzlösung können die Kinetik und die thermodynamischen Eigenschaften des Entkeimungssystems an die Eigenschaften der zu entkeimenden Wässer und deren Wasserhärte angepasst werden.

Durch die Behandlung der oxidierten Edelmetalloberfläche mit der Salzlösung wird direkt auf der elementaren Silberdrahtoberfläche eine hochporöse, amorphe Edelmetallsalzschicht im subµ-Bereich gebildet, aus der, inhomogen verteilt, kristalline, bis auf den Silberdraht hinabreichende Bereiche herauswachsen.

Als Kationen können in der Salzlösung beispielsweise Alkali- oder Erdalkalikationen eingesetzt werden. Auf den Einsatz von Ammoniumverbindungen als Kationen und Sulfidionen als Anionen sollte sowohl in der Salzlösung, als auch in der Oxidationslösung verzichtet werden, da diese die Edelmetallionen komplexieren beziehungsweise blockieren.

Die Verweilzeit im Salzbad beträgt wenige Minuten bis einige Tage.

Um eine rasche Bildung der gewünschten schwerlöslichen Edelmetallsalze oder des Gemischs von Edelmetallsalzen mit unterschiedlichem Löslichkeitsprodukt auf der Oberfläche zu erreichen, empfiehlt es sich, die gewünschten Anionen in der Salzlösung in hohen Konzentrationen bereitzustellen, das heißt, in Wasser leichtlösliche Salze in hoher Konzentration, gegebenenfalls gar in gesättigter Lösung, einzusetzen. Auf jeden Fall sollte die Gesamtsalzkonzentration wenigstens 0,1 Gew.% betragen. Nach Entnahme des Systems aus dem Salzbad ist es zur Entkeimung einsatzbereit.

Vorzugsweise sollte die Behandlung in der sauren Lösung das Abschrecken mit Leitungswasser und/oder die Behandlung in der Salzlösung bei einer Temperatur zwischen 10° und 130° C, vorzugsweise bei weniger als 80° C und besonders bevorzugt bei 20° C ± 5° C, erfolgen.

Durch die erfindungsgemäße Behandlung der Edelmetalle beziehungsweise Edelmetallionen-oberfläche stellt sich in dem zu entkeimenden Wasser über die Wechselwirkung der Edelmetallsalze auf der Oberfläche und den im Wasser befindlichen Gegenionen selbstständig die jeweilige Gleichgewichtskonzentration ein. Das Entkeimungssystem ist somit selbstregulierend.

Über die chemische Zusammensetzung der Oberfläche, also über den jeweiligen Anteil der unterschiedlich schwer löslichen Edelmetallverbindungen auf der Oberfläche, lässt sich darüber hinaus die maximal abgegebene Edelmetallmenge so einjustieren, dass die gültigen Grenzwerte sicher eingehalten werden. Das Entkeimungssystem lässt sich somit an unterschiedliche Wasserqualitäten und Grenzwerte adaptieren.

Das Trägermaterial übernimmt nicht nur mechanische Aufgaben. Es ist vielmehr auch Wirkstoff-Depot. Aufgrund der hohen Edelmetallmenge im Bulk lassen sich so problemlos Standzeiten von mehreren Jahren bei gleichbleibender Wirkung erzielen, so dass eine Langzeitdepotwirkung erreicht wird.

Dadurch, dass das erfindungsgemäße System das Redoxpaar Edelmetall/Edelmetallion, insbesondere Ag/Ag⁺ mit dem Edelmetall, insbesondere Silber, im Überschuss bereitstellt, ist das System weitgehend unempfindlich gegen oxidierende oder reduzierende Substanzen und gegen Belichtung.

Im allgemeinen liegt die Temperatur der sauren Lösung, der Salzlösung und des Leitungswassers bei der Behandlung zwischen 10° C und 130° C, vorzugsweise bei höchstens 80° C und besonders bevorzugt bei 20° C ± 5° C.

Erfindungsgemäß wird als Basismaterial ein Gestrick, Gewirk oder Gewebe aus Edelmetallfäden mit Kohlenstofffäden oder Kunststofffäden eingesetzt. Das Edelmetall-Kunststoff-Gewebe, -Gestrick oder -Gewirk wird nach dem Strick- oder Webvorgang thermisch in Kohlenstoff umgewandelt. Diese Edelmetall/Kohlenstoff-Basismaterialen wirken nicht nur antimikrobiell, sondern auch adsorptiv.

Die erfindungsgemäß behandelten Silberdrähte beziehungsweise -fäden können in einer weiteren Ausführungsform auch in Form eines Geflechtes eingesetzt werden, beispielsweise in Perlatoren, zum Schutz von Oberflächen, für Keimbarrieren oder in Ionengeneratoren.

Eine ungeheure Vielzahl von Anwendungsmöglichkeiten ergeben sich aus dem Einsatz der erfindungsgemäß behandelten Silberpulver.

Diese Pulver können beispielsweise in aus der Kunststofftechnik der, Oberflächentechnik oder der Pulvertechnologie bekannten Verfahren verarbeitet werden:

So ist es beispielsweise möglich, die behandelten Edelmetallpulver in eine Kunststoffmatrix einzubetten (Blends, Co-Extrusion, Co-Laminierung). Auch können die Pulver zur Beschichtung von Oberflächen eingesetzt werden, beispielsweise um das Material zu schützen, zu entkeimen oder von Keimen freizuhalten. Die Beschichtung kann beispielsweise durch Tauchen, Sprühen oder Pulverlackierung erfolgen.

Die behandelten Pulver können als solche oder als Gemisch mit metallischen Pulvern oder nichtmetallischen Pulvern geformt werden, um einen porösen antimikrobiellen Formkörper bereitzustellen. Als nichtmetallische Pulver können beispielsweise Aktivkohle, Zeolithe, Silicagel etc. eingesetzt werden. Die Formgebung kann mit bekannten Pressverfahren wie CIP oder HIP oder Schlickerguss etc. erfolgen.

Auch lassen sich Drähte, Garne oder Fäden, aber auch Schäume mit dem erfindungsgemäß behandelten Pulver beispielsweise durch Co-Extrusion oder Tauchen beschichten oder imprägnieren.

Prinzipiell ist es möglich, alle denkbaren Geometrien und Materialien mit dem erfindungsgemäß behandelten Edelmetallpulver, insbesondere durch Tauchen, zu beschichten. So können erfindungsgemäß behandelte Edelmetallpulver umfassende Sinterkörper bereitgestellt werden.

Die porösen Formkörper aus dem erfindungsgemäß behandelten Pulver oder die Pulver mit Zusätzen von Metall - oder Nichtmetallpulvern können in Perlatoren oder als Metallfilter eingesetzt werden.

Mit dem erfindungsgemäßen Entkeimungssystem ist es möglich, Trinkwasser zuverlässig zu entkeimen beziehungsweise nachhaltig vor Rückverkeimung zu schützen. Durch die erfindungsgemäße Oberflächenbeschaffenheit wird erreicht, dass nur minimale Silberionenkonzentrationen, benötigt werden. So werden die geltenden Richtwerte sicher eingehalten. Die Einstellung dieser geringen, aber zur zuverlässigen Wirkung ausreichenden Konzentrationen erfolgt automatisch über chemische Gleichgewichtseinstellungen. Die Absolutmenge an eingesetztem Material bestimmt die Standzeit, die deutlich über einem bis mehrere Jahre liegt. Durch die Selbstregulierung und - erneuerung kann auf den Einsatz einer Mess-, Steuer- und Regeltechnik verzichtet werden.

Das erfindungsgemäße Entkeimungssystem kann an jeder beliebigen Stelle in den Wasserkreislauf eingebracht werden. Verschiedene typische Anwendungen des erfindungsgemäßen Entkeimungssystems sind in den nachfolgenden Figuren 1 bis 3 dargestellt. Es zeigen:
- Figur 1: eine schematische Darstellung von Hausanlagen und Wasserfiltern mit dem erfindungsgemäßen Entkeimungssystem,
- Figur 2: Anwendungsbeispiele in einem geschlossenen und quasigeschlossenen System und
- Figur 3: Anwendungsbeispiele für die "Dritte Welt".

Das Material selbst befindet sich entweder in separaten Kartuschen, die über übliche Verbindungstechnologien in die Leitung integriert werden oder es befindet sich direkt in den Filtern oder in den Tanks. Als Basismaterial wird, abhängig vom geplanten Einsatz, bevorzugt Silberdraht, Silberwolle beziehungsweise ein mit Silberdraht durchwobenes Kunststoffgewebe eingesetzt.

Dieses erfindungsgemäße Entkeimungssystem kann beispielsweise in Hausanlagen zur Nachbehandlung von Stadtwasser, zur Entkeimung von Luftbefeuchtern, Warmwasser- und Tanksystemen oder geschlossenen Systemen zur dezentralen Trinkwasser-Desinfektion in der "Dritten Welt", eingesetzt werden:

### a) Einsatz oberflächenbehandelter Silber-/Kunststoffgewebe bzw. Silberwollen in Hausanlagen zur Nachbehandlung von Stadtwasser

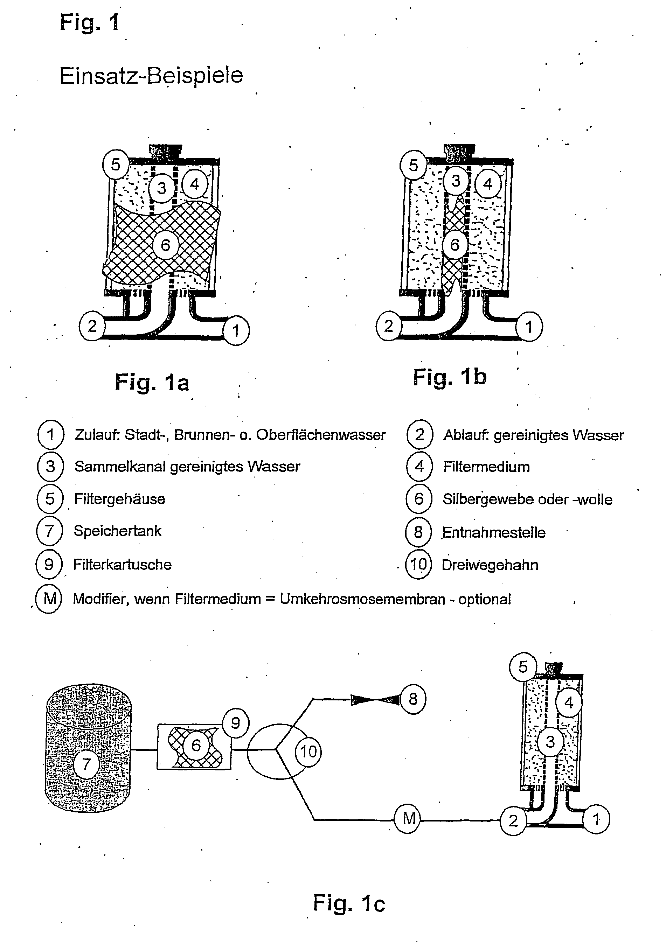

Aktivkohlefilter für den häuslichen Bereich entfernen prinzipbedingt das zum Schutz gegen Rückverkeimung eingesetzte Chlor. Das führt dazu, dass die Filter in kurzer Zeit von Mikroorganismen besiedelt werden, welche zu einer starken bakteriologischen Belastungen des Trinkwassers führen. Um dies zu verhindern, kann ein erfindungsgemäßes Entkeimungssystem, beispielsweise Silbergewebe oder -wolle oder Silber/Kunststoffgewebe um das Filtermedium (4) (Filtermembran beispielsweise den Filterblock (gepresste Aktivkohleblöcke)) gewickelt werden, was eine direkte Besiedlung der Filtermedien verhindert (Figur 1 a). Alternativ kann das Entkeimungssystem direkt in die Sammelleitung des Produktwassers (3) in Figur 1 b (Umkehrosmose, Aktivkohle - sowohl gepresste Blöcke, als auch Granulat) eingebracht werden. So behandeltes Wasser ist keimfrei und aufgrund der gelösten Edelmetallionen gegen Rückverkeimung gesch ützt.

Bei Systemen mit Zwischenspeichertank (7) ist es verteilhaft, das Entkeimungssystem zwischen Tank (7) und Dreiwegehahn (10) zu schalten, wie in Figur 1 c dargestellt ist.

Abhängig von der Ausgangsbelastung und den allgemeinen Situationen können die Möglichkeiten kombiniert werden. In der Regel genügt allerdings eine der drei Möglichkeiten, um bei Stadtwasser-Nachbehandlungsanlagen oder bei Brunnenwasser einen ausreichenden Schutz zu gewährleisten. Bei Anlagen ohne Tank kommen insbesondere die den Figuren 1 a, b gezeigten Varianten zum Einsatz, während bei Anlagen mit Tank bevorzugt die in Figur 1 c dargestellte Variante beziehungsweise deren Kombination eingesetzt wird.

### b) Einsatz oberflächenbehandelter Silber-/Kunststoffgewebe beziehungsweise Silberwollen in Luftbefeuchtern, Warmwasser- und Tanksystemen oder in geschlossenen Systemen

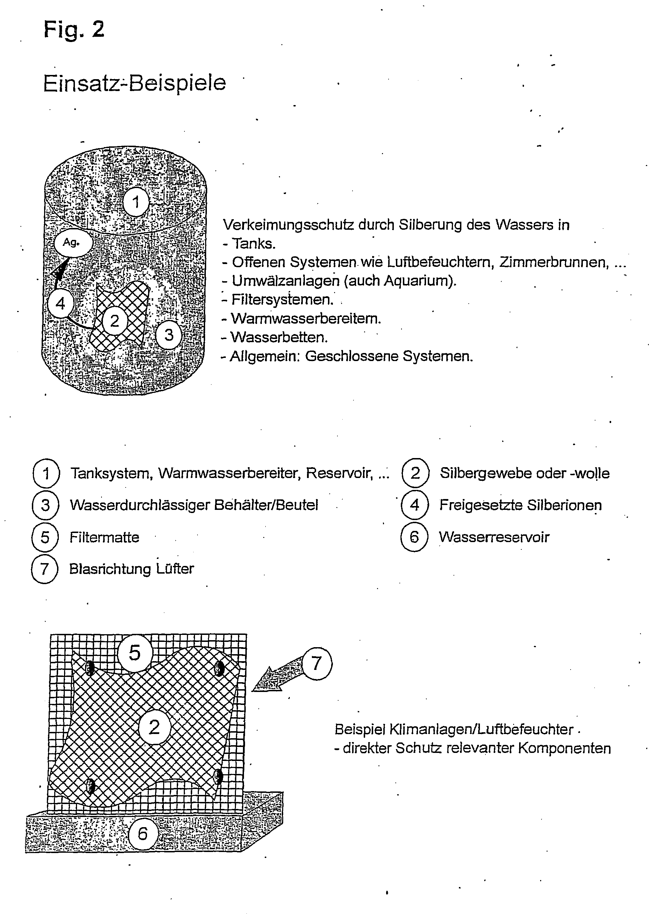

In den in den Figuren 2 a und b dargestellten Anwendungsbeispielen wird das erfindungsgemäße Entkeimungssystem überwiegend in geschlossenen Systemen (Figur 2 a) oder in quasi-geschlossenen Systemen wie Speichertanks (Batch-Betrieb) eingesetzt, wobei es vorzugsweise direkt in den Tank eingebracht wird.

In Luftbefeuchtern, Klimaanlagen, Warmwassertanks oder Untertischboilern oder Wassertanks (Camping) wird mit der Zeit ein starkes Anwachsen der Mikrobiologie beobachtet. Dies führt zu Gesundheitsgefährdungen (Luftbefeuchter, Wassertanks etc.) Dies kann durch den Einsatz des erfindungsgemäßen Systems mit Silber-/Kunststoffgewebe als Basismaterial beziehungsweise durch Einsatz eines erfindungsgemäßen Systems mit Silberwolle als Basismaterial verhindert werden (Figur 2). Hierbei können die Materialien auf Silberbasis entweder direkt beziehungsweise in wasserdurchlässigen Gehäusen eingebracht werden (Figur 2 a). Bei entsprechenden Luftbefeuchterarten kann das erfindungsgemäße System auf Silber-/Kunststoffgewebebasis auch direkt auf die Filtermatten (5) in Figur 2 b gelegt werden. Zum Schutz gegen Legionella können zusätzlich behandelte Cu-Fäden eingewoben werden.

### c) Einsatz oberflächenbehandelter Silber-/Kunststoffgewebe bzw. Silberwollen zur dezentralen Trinkwasserdesinfektion - "Dritte Welt"

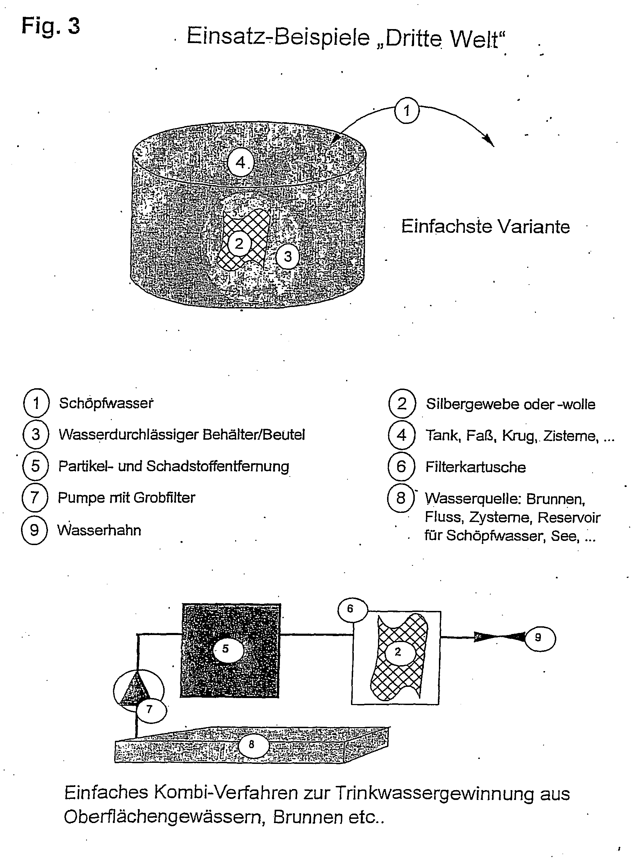

Hygienisch einwandfreies Trinkwasser ist in der "Dritten Welt" nur eingeschränkt verfügbar. Angesichts fehlender Infrastrukturen muss eine einfache, dezentrale Trinkwasseraufbereitung zur Verfügung gestellt werden. Hierfür können erfindungsgemäß behandelte Silber-/Kunststoffgewebe beziehungsweise Silberwollen vorteilhaft eingesetzt werden. Im einfachsten Fall wird hierzu das erfindungsgemäß behandelte Silber-/Kunststoffgewebe beziehungsweise die Silberwolle in den entsprechend bestückten Vorratsbehälter - Wassergefäß - gegeben und die Gefäße anschließend mit dem geschöpften Brunnen- oder Flusswasser aufgefüllt (Figur 3 "einfachste Variante"). Bei entsprechenden Einwirkzeiten des Entkeimungssystems kann auch mikrobiell hoch belastetes Wasser entkeimt werden. Damit eignen sich die erfindungsgemäßen Entkeimungssysteme nicht nur für die Verhinderung einer Rückverkeimung, sondern auch zur Aufbereitung von Trinkwasser. In einfachen Fällen genügt das mehrstündige Aufbewahren von geschöpftem Oberflächen- oder Brunnenwasser in Flaschen, Kanistern oder anderen Gefäßen.

Wird Regenwasser in Zisternen gesammelt, dann kann man eine entsprechend behandelte Menge Silber-/Kunststoffgewebe beziehungsweise Silberwolle in die Zisterne beziehungsweise in den Auslauf (sofern vorhanden) einbringen (Figur 3).

Die Trinkwasserdesinfektion mit den erfindungsgemäß behandelten Silber-/Kunststoffgeweben beziehungsweise Silberwollen kann mit anderen Technologien kombiniert werden. So stellt diese Technik zusammen mit einer Wasserpumpe und einem einfachen Filtersystem, wie zum Beispiel einer Kombination aus Sand- und Aktivkohlefilter, eine vollständige, einfach zu handhabende Wasseraufbereitungsanlage dar (Figur 3 "einfaches Kombiverfahren"). Abhängig von den technischen Gegebenheiten sind weitere Kombinationen realisierbar,

Die Erfindung wird nachfolgend anhand von Ausführungsbeispielen beschrieben.

### 1. Ausführungsbeispiel: Herstellung eines Entkeimungssystems aus Silberdraht

Ein Silberdraht mit einem Durchmesser von 0,05 bis 0,5 mm wird zunächst platt gewalzt, um die Oberfläche zu vergrößern. Anschließend wird er etwa drei bis fünf Sekunden in einer sauren wässrigen Lösung, die als Säure eine Verbindung mit Stickstoff in der Oxidationsstufe + 5 und/oder Schwefel in der Oxidationsstufe + 6 und/oder Sauerstoff in der Oxidationsstufe - 1 umfasst, behandelt, mit Leitungswasser abgespült und in einer Cl⁻ umfassenden Lösung über Nacht gelagert.

Die Herstellung des Entkeimungssystems kann auch in einem kontinuierlichen Verfahren erfolgen. Hierbei wird Silberdraht, gegebenenfalls platt gewalzt, der sich auf einer Spule befindet, kontinuierlich abgewickelt und durch die saure wässrige Lösung, vorzugsweise eine konzentrierte Lösung einer Oxosäure der Elemente der V - VII Hauptgruppe der zweiten und dritten Periode in deren höchsten Oxidationsstufe, circa drei bis fünf Sekunden hindurchgeführt.

In einem weiteren Bad mit Leitungswasser wird der oxidierte Silberdraht "abgeschreckt" und anschließend in eine Salzlösung überführt.

### 2. Ausführungsbeispiel: Herstellung eines Entkeimungssystems aus einem Silber/Polypropylengewebe

Ein Gewebe aus Polypropylenfäden mit Silberfäden wird circa 3 bis 5 Sekunden in der sauren Lösung und anschließend in Leitungswasser und der Salzlösung gemäß Ausführungsbeispiel 1 behandelt, wodurch die Oberfläche der Silberfäden im wesentlichen oxidiert wird. Ein Teil der Ag⁺-Ionen werden in das Polypropylen infiltriert, so dass in diesem System zusätzlich zu den Ag⁺-Ionen auf der Silberoberfläche auch leicht verfügbare Ag⁺ in dem PP-Gewebe zur Verfügung stehen und die wirksam entkeimende Oberfläche im Vergleich zu reinem Silberdraht vergrößert ist.

### 3. Ausführungsbeispiel: Herstellung eines Entkeimungssystems mit Kohlefaser/Silbergewebe

Ein Kunststofffaden-/Silberdraht-Mischgewebe wird thermisch in ein Kohlefaser/Silberdraht-Mischgewebe umgesetzt. Anschließend wird gemäß dem Ausführungsbeispiel 1 verfahren.

### 4. Ausführungsbeispiel: Herstellung eines Entkeimungssystems aus behandeltem Silberpulver

Anstelle des Silberdrahts gemäß Ausführungsbeispiel 1 wird kommerzielles Ag-Pulver eingesetzt, das zunächst deglomeriert und dann im Bereich von Sekunden bis Minuten in die saure wässrige Lösung gegeben wird. Anschließend wird abgetrennt, gewaschen und das gewaschene Pulver über Nacht in der Cl⁻ umfassenden Lösung gelagert.

### 5. REM-Aufnahme der Oberfläche eines Entkeimungssystems

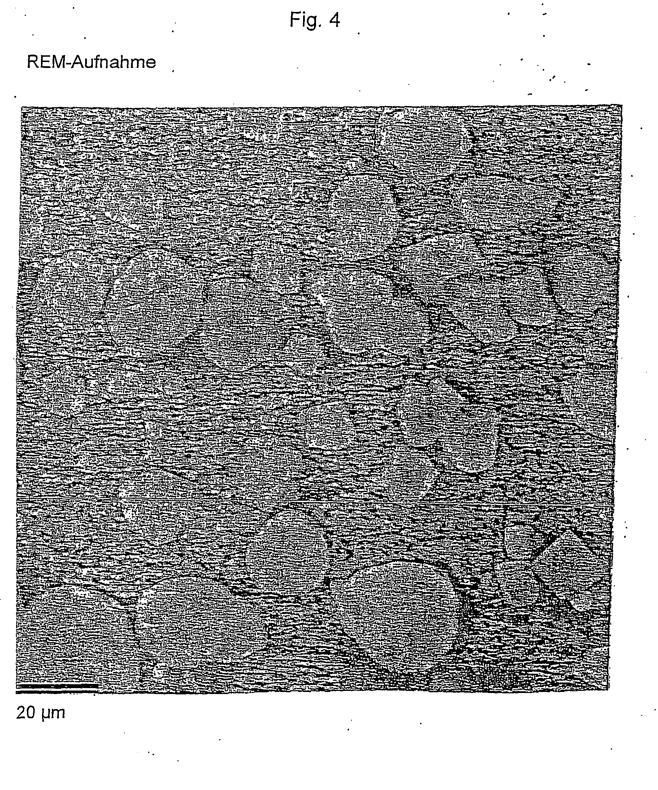

Um die Silberoberfläche mittels REM-Aufnahmen zu untersuchen, wurde aus einem gemäß Ausführungsbeispiel 2 hergestellten Entkeimungssystem (Polypropylen/Silberfaden; Salzbad: Kochsalzlösung) Polypropylen entfernt und der verbleibende Silberfaden mit der oxidierten Oberfläche untersucht. Die REM-Aufnahme ist in **Figur 4** dargestellt. Der Balken im unteren Bereich der REM-Aufnahme entspricht 20 µm.

Die Aufnahme zeigt eine dünne, amorphe und poröse Oberflächenschicht, deren Dicke voraussichtlich im Submikrometer-Bereich liegen dürfte. Bei den dunklen Bereichen unter der amorphen OberflächenSchicht dürfte es sich um den blanken Silberdraht handeln. Die REM-Aufnahme zeigt weiterhin kristalline Silber-Verbindungen (zum Beispiel AgCl, Ag₂CO₃ etc.) auf der Oberfläche.

Das erfindungsgemäße Entkeimungssystem zeichnet sich somit durch eine heterogene, extrem große Oberfläche aus, und es sind Bereicheunterschiedlicher Aktivität vorhanden (amorphe und kristalline Bereiche). Die Redox-Aktivitäten der im direkten Kontakt zur blanken Silberoberfläche stehenden amorphen beziehungsweise kristallinen Oberflächenregionen sind ebenfalls unterschiedlich. Hierdurch lässt sich insgesamt die Einstellung des Gleichgewichts (Kinetik) als auch die Gleichgewichtskonzentration selbst (Thermodynamik) steuern.

## Patentansprüche

1. Verwendung eines Systems zur Entkeimung von Trinkwasser, **dadurch gekennzeichnet, dass** das System ein Basismaterial umfasst, das ein massiver Körper aus Edelmetallen oder eine Edelmetall umfassende Legierung, ein Edelmetalldraht, Edelmetallwolle, Edelmetallgestrick oder -gewebe ist und die Oberfläche des Basismaterials wenigstens teilweise oxidiert ist und das oxidierte Edelmetall an der Oberfläche als schwerlösliches Edelmetallsalz oder als Gemisch von Edelmetallsalzen mit unterschiedlicher Löslichkeit vorliegt und bei der Entkeimung von der Oberfläche abgegebene Edelmetallionen aus dem Edelmetall des Basismaterials nachgeliefert werden, wobei das Edelmetall Silber ist, die Oberfläche mikroporös und amorph ist und die Oberfläche kristallines AgCl umfasst.

2. Verwendung eines Systems Anspruch 1, **dadurch gekennzeichnet, dass** die kristallinen und amorphen Edelmetallsalze im direkten Kontakt zueinander und im direkten Kontakt zur blanken Silberoberfläche stehen, so dass sich insgesamt folgende Regionen unterschiedlicher Redoxaktivität und Löslichkeit ausbilden: i) über Poren der amorphen Schicht zugängliche reine Silberoberfläche, ii) amorphe Edelmetallsalzschicht, iii) kristalline Edelmetallsalzschicht, iv) Kontaktzone Silber - amorphe Edelmetallsalzschicht, v) Kontaktzone Silber - kristalline Edelmetallsalzschicht, vi) Kontaktzone amorphe Edelmetallsalzschicht - kristalline Edelmetallsalzschicht, vii) Kontaktzone Silber - amorphe Edelmetallsalzschicht - kristalline Edelmetallsalzschicht.

3. Verwendung eines Systems nach einem der vorangegangenen Ansprüche, **dadurch gekennzeichnet, dass** es zusätzlich Funktionsfäden wie Aktivkohle oder Sensorfäden umfasst.

4. Verwendung eines Systems nach einem der vorangegangenen Ansprüche, **dadurch gekennzeichnet, dass** das Basismaterial ein Edelmetallpulver ist und das behandelte Edelmetallpulver zur Beschichtung von Oberflächen, als solches oder im Gemisch mit metallischen und/oder nichtmetallischen Pulvern in antimikrobiellen Formkörpern gepresst oder in bekannten Verfahren der Pulvertechnologie eingesetzt wird.

## Claims

1. Use of a system for sterilizing drinking water, **characterized in that** it comprises a base material, which base material is a solid body made of noble metal or an alloy comprising noble metal, a noble metal wire, noble metal wool, knitted noble metal fabric or woven noble metal fabric, the surface of the noble metal is at least in part oxidized and the oxidized noble metal is present on the surface as slightly soluble noble metal salt or as a mixture of noble metal salts of different solubility, and during the sterilization, noble metal ions released from the surface are replenished from the noble metal of the base material, the noble metal being silver and the surface being microporous and amorphous and the surface comprising crystalline AgCl.

2. Use of a system as claimed in claim 1, **characterized in that** the crystalline and amorphous noble metal salts are in direct contact with one another and in direct contact with the bare silver surface, so that, overall, the following regions of differing redox activity and solubility form:
i) pure silver surface accessible via pores of the amorphous layer,
ii) amorphous noble metal salt layer,
iii) crystalline noble metal salt layer,
iv) contact zone silver - amorphous noble metal salt layer,
v) contact zone silver - crystalline noble metal salt layer,
(vi) contact zone amorphous noble metal salt layer - crystalline noble metal salt layer,
(vii) contact zone silver - amorphous noble metal salt layer - crystalline noble metal salt layer.

3. Use of a system as claimed in one of the preceding claims, **characterized in that** it additionally comprises functional threads such as activated carbon or sensory threads.

4. Use of a system as claimed in one of the preceding claims, **characterized in that** the base material is a noble metal powder and the treated noble metal powder is used for coating surfaces, as such or in a mixture with metallic and/or nonmetallic powders compressed in antimicrobial shaped bodies, or in known processes of powder technology.

## Revendications

1. Utilisation d'un système de stérilisation d'eau potable,
**caractérisée en ce que**
le système comprend un matériau de base qui est un corps massif en métal précieux ou un alliage comprenant un métal précieux, un fil en métal précieux, une laine en métal précieux, un tricot ou un tissu en métal précieux,
**en ce qu'**au moins une partie de la surface du matériau de base est oxydée et
**en ce que** le métal précieux oxydé présent à la surface a la forme d'un sel peu soluble de métal précieux ou d'un mélange de sels de métal précieux de différentes solubilités et
**en ce que** lors de la stérilisation, les ions de métal précieux délivrés par la surface proviennent du métal précieux du matériau de base, le métal précieux étant l'argent, la surface étant microporeuse et amorphe et la surface contenant de l'AgCl cristallin.

2. Utilisation d'un système selon la revendication 1, **caractérisée en ce que** les sels cristallins de métal précieux et les sels amorphes de métal précieux sont en contact direct les uns avec les autres et en contact direct avec la surface nue d'argent, de telle sorte qu'il se forme globalement les zones suivantes d'activités redox et de solubilités différentes :
i) une surface d'argent pur accessible à travers les pores de la couche amorphe,
ii) une couche de sel amorphe de métal précieux,
iii) une couche de sel cristallin de métal précieux,
iv) une zone de contact entre l'argent et la couche de sel amorphe de métal précieux,
v) une zone de contact entre l'argent et la couche de sel cristallin de métal précieux,
vi) une zone de contact entre la couche de sel amorphe de métal précieux et la couche de sel cristallin de métal précieux et
vii) une zone de contact entre l'argent, la couche de sel amorphe de métal précieux et la couche de sel cristallin de métal précieux.

3. Utilisation d'un système selon l'une des revendications précédentes, **caractérisée en ce que** le système comprend en supplément des fils fonctionnels, par exemple en charbon actif, ou des fils de détection.

4. Utilisation d'un système selon l'une des revendications précédentes, **caractérisée en ce que** le matériau de base est une poudre de métal précieux et **en ce que** la poudre de métal précieux traitée est utilisée pour revêtir des surfaces, seule ou en mélange avec des poudres métalliques et/ou des poudres non métalliques comprimées en corps antimicrobiens façonnés, ou est utilisée dans des procédés connus de la technologie des poudre
